# EUROPEAN PATENT APPLICATION

(11) **EP 4 455 289 A1**
(43) Date of publication of application: **30.10.2024**
(21) Application number: 22911296.6
(22) Date of filing: 21.12.2022
(51) Int. Cl.: C12N 15/56, C11D 7/42, C12N 9/28, C12N 9/52

(54) **AMYLASE-INCORPORATED CLEANING AGENT COMPOSITION**

(30) Priority: 24.12.2021 JP 2021211185
(71) Applicant: Kao Corporation, Tokyo 103-8210 (JP)
(72) Inventor: SHAKU, Mao, Wakayama-shi, Wakayama 640-8580 (JP); HIOKI, Takahiro, Wakayama-shi, Wakayama 640-8580 (JP); KAWAHARA, Akihito, Wakayama-shi, Wakayama 640-8580 (JP)
(74) Representative: Vossius & Partner Patentanwälte Rechtsanwälte mbB
(86) International application number: PCT/JP2022/047187
(87) International publication number: WO 2023/120594

(57) **Abstract**

Provided is an α-amylase-containing cleaning composition which has high amylolytic activity at low temperatures and also has high stability even in a protease-containing composition. A cleaning composition comprising an α-amylase mutant and protease, wherein the α-amylase mutant is a mutant of a parent α-amylase and comprises one or more amino acid residue substitutions at positions selected from the group consisting of positions corresponding to positions E187 and S241 in the amino acid sequence of SEQ ID NO: 2, and further one or more amino acid residue substitutions at positions selected from the group consisting of positions corresponding to positions N192, H240, and K278, wherein the parent α-amylase or α-amylase mutant has at least 85% sequence identity to the amino acid sequence of SEQ ID NO: 4.

## Description

### Field of the Invention

The present invention relates to a cleaning composition containing an **α**-amylase and protease.

### Background of the Invention

**α**-amylases are used in a wide range of industries, such as starch, brewing, textiles, pharmaceuticals, and food, are known to have suitability for containing in cleaning agents, and are contained in dishwashing cleaning agents for automatic dishwashers and clothing cleaning agents as components removing starch stains.

Known **α**-amylases useful for cleaning agents are Bacillus sp. KSM-1378 (FERM BP-3048) strain-derived **α-**amylase AP1378 (Patent Literature 1), Termamyl and Duramyl (registered trademarks), which are *Bacillus licheniformis-derived* **α**-amylases, Bacillus sp. DSM12649 strain-derived **α**-amylase AA560 (Patent Literature 2), Bacillus sp. SP722 strain-derived **α**-amylase SP722 (SEQ ID NO: 4 of Patent Literature 3), Cytophaga-derived **α-**amylase CspAmy2 (Patent Literature 4), and the like. With regard to these **α**-amylases, mutants modified to improve their functions for specific applications, for example, mutants with enhanced stability in cleaning agents, have been reported (Patent Literature 5).

In recent years, from the viewpoint of environmental protection and cleaning cost reduction, it is important to reduce temperatures in dishwashing and laundry washing, particularly in laundry washing in laundries. In addition, shortening of the cleaning time is also desired. However, optimal temperatures of most enzymes, including amylases, are higher than temperatures generally set for low-temperature cleaning. For this reason, it is difficult to completely remove many starch stains.

Under such circumstances, the present applicant found an α-amylase which maintains cleaning performance and amylolytic activity even at low temperatures, and has a high stain removal effect (Patent Literature 6).

Since many stains containing starch also contain protein components, it is known that the combined use of α-amylases and protease produces an additive and synergistic cleaning effect. Such cleaning agents containing an **α**-amylase and protease are particularly useful for cleaning at low temperatures where the decrease in detergency is an issue. On the other hand, the combined use of protease causes the problem in that the stability of amylase in the cleaning agent is impaired.

[Patent Literature 1] WO 94/26881
[Patent Literature 2] WO 00/60060
[Patent Literature 3] WO 06/002643
[Patent Literature 4] WO 2014/164777
[Patent Literature 5] WO 98/044126
[Patent Literature 6] JP 2021-112712

### Summary of the Invention

The present invention relates to the following:
A cleaning composition comprising an **α**-amylase mutant and protease, wherein the **α**-amylase mutant is a mutant of a parent **α**-amylase and comprises one or more amino acid residue substitutions at positions selected from the group consisting of positions corresponding to positions E187 and S241, and further one or more amino acid residue substitutions at positions selected from the group consisting of positions corresponding to positions N192, H240, and K278 in the amino acid sequence of SEQ ID NO: 2,
wherein the parent **α**-amylase or **α**-amylase mutant has at least 85% sequence identity to the amino acid sequence of SEQ ID NO: 4.

### Brief Description of Drawing

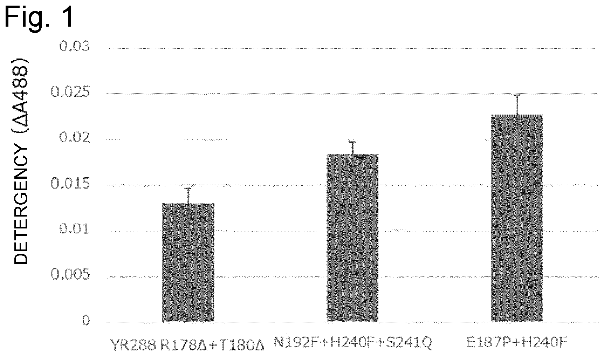

[Fig. 1] Fig. 1 shows the evaluation of amylase detergency after storage in a protease-containing cleaning composition.

### Detailed Description of the Invention

The present invention provides an **α**-amylase-containing cleaning composition which exhibits high amylolytic activity at low temperatures and exhibits high stability even in a protease-containing composition.

The present inventors found that a specific **α-**amylase mutant having excellent cleaning performance and stability even at low temperatures maintains its performance even when combined with protease, and is useful as an enzyme to be contained into cleaning agents suitable for low-temperature cleaning.

The present invention can provide a cleaning composition containing an **α**-amylase and protease, stably exhibiting an excellent starch stain removal effect even at low temperatures, and having high storage stability.

In the present invention, "amylase" (EC3.2.1.1; **α**-D-(1→4)-glucan glucanohydrolase) refers to a group of enzymes that catalyze the hydrolysis of starch and other linear or branched 1,4-glycoside oligosaccharides or polysaccharides. The **α**-amylase activity can be determined by measuring the amount of reducing ends produced by the enzymatic degradation of starch. The determination method is not limited thereto; for example, the **α**-amylase activity can also be determined by measuring the release of dye by the enzymatic degradation of dye-crosslinked starch, such as Phadebas (Soininen, K., M. Ceska, and H. Adlercreutz. "Comparison between a new chromogenic α-amylase test (Phadebas) and the Wohlgemuth amyloclastic method in urine." Scandinavian journal of clinical and laboratory investigation 30.3 (1972): 291-297.).

In the present invention, the identity of amino acid sequences or nucleotide sequences is calculated by the Lipman-Pearson method (Science, 1985, 227: 1435-1441). Specifically, the identity is calculated by analysis using a homology analysis (Search homology) program of genetic information processing software GENETYX Ver. 12 at a unit size to compare (ktup) of 2.

The "amino acid residues" herein refer to 20 amino acid residues constituting a protein, i.e., alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

Amino acid positions and mutants are herein denoted as shown below using the officially recognized IUPAC oneletter amino acid abbreviations.

An amino acid at a predetermined position is denoted as [amino acid, position]. For example, threonine at position 226 is denoted as "T226".

"Substitution" of an amino acid is denoted as [original amino acid, position, substituted amino acid]. For example, substitution of threonine at position 226 with alanine is expressed as "T226A".

"Deletion" of an amino acid is denoted as [original amino acid, position, Δ]. For example, deletion of serine at position 181 is expressed as "S181Δ".

"Insertion" of an amino acid is denoted as [original amino acid, position, original amino acid, inserted amino acid]. For example, insertion of lysine after glycine at position 195 is expressed as "G195GK". Insertion of multiple amino acids is denoted as [original amino acid, position, original amino acid, inserted amino acid 1, inserted amino acid 2; and the like]. For example, insertion of lysine and alanine after glycine at position 195 is expressed by "G195GKA".

Mutants including multiple modifications are denoted by using the addition symbol ("+"). For example, "R170Y+G195E" represents substitution of arginine at position 170 with tyrosine and substitution of glycine at position 195 with glutamic acid, respectively.

When it is possible to introduce different modifications at one position, the different modifications are divided by the slash ("/"). For example, "R170Y/E" represents substitution of arginine at position 170 with tyrosine or glutamic acid.

The "operable linkage" between a regulatory region, such as a promoter, and a gene herein means that the gene and the regulatory region are linked so that the gene can be expressed under the control of the regulatory region. Procedures for the "operable linkage" between the gene and the regulatory region are well known to a person skilled in the art.

The "upstream" and "downstream" relating to a gene herein refer to the upstream and downstream in the transcription direction of the gene. For example, "a gene located downstream of a promoter" means that the gene is present on the 3' side of the promoter in the DNA sense strand, and the upstream of a gene means a region on the 5' side of the gene in the DNA sense strand.

The term "original" used herein for a function, property, or trait of a cell is used to indicate that the function, property, or trait is inherent in the cell. In contrast, the term "foreign" is used to describe a function, property, or trait that is introduced from outside the cell, rather than being inherent in the cell. For example, a "foreign" gene or polynucleotide is a gene or polynucleotide introduced into the cell from outside. The foreign gene or polynucleotide may be derived from an organism of the same species as the cell into which the foreign gene or polynucleotide is introduced, or from an organism of a different species (i.e., heterologous gene or polynucleotide).

The **α**-amylase mutant contained in the cleaning composition of the present invention (hereinafter also referred to as the "the mutant of the present invention") is a mutant of a parent **α**-amylase, containing one or more amino acid residue substitutions at positions selected from the group consisting of positions corresponding to positions E187 and S241, and further one or more amino acid residue substitutions at positions selected from the group consisting of positions corresponding to positions N192, H240, and K278 in the amino acid sequence of SEQ ID NO: 2.

That is, the mutant of the present invention means a polypeptide having **α**-amylase activity in which a plurality of amino acid residues at predetermined positions are substituted with other amino acid residues in amino acids constituting the parent **α**-amylase. The substitution of amino acid residues at such predetermined positions is a modification intended for enhancing the cleaning performance and/or stability in protease-containing cleaning agents. Therefore, such a mutant has enhanced cleaning performance and/or stability in comparison with the parent **α**-amylase.

In the mutant of the present invention, modification sites (mutation positions) of amino acid residues are one or more positions selected from the group consisting of positions corresponding to positions E187 and S241, and further one or more positions selected from the group consisting of positions corresponding to positions N192, H240, and K278 in the amino acid sequence of SEQ ID NO: 2.

In addition to the above combinations of modifications, the mutant of the present invention may further contain one or more amino acid residue substitutions at positions selected from the group consisting of positions corresponding to positions F205, R211, and M199 in the amino acid sequence of SEQ ID NO: 2.

Furthermore, the mutant of the present invention may also contain one or more amino acid residue substitutions at positions selected from the group consisting of positions corresponding to positions G5, S38, Q96, W186, E257, F259, S284, H295, Y296, N303, T320, Y360, W408, L429, V430, G433, A434, W439, N471, G476, and G477 in the amino acid sequence of SEQ ID NO: 2.

The amino acid sequence of SEQ ID NO: 2 constitutes **α**-amylase YR288, and the mutation positions in the mutant of the present invention are numbered based on the amino acid numbers of the amino acid sequence.

YR288 is a protein registered as WP_100346362.1 in the NCBI protein sequence database, and is a protein specified by the present applicant as an α-amylase having high amylolytic activity and cleaning performance at low temperatures (JP-A-2020-121626).

The "corresponding position" on the amino acid sequence can be determined by aligning the target sequence and the reference sequence (the amino acid sequence of SEQ ID NO: 2 in the present invention) so as to give maximum homology. Alignment of the amino acid sequences can be performed using known algorithms, the procedure of which is known to a person skilled in the art. For example, alignment can be performed by using the Clustal W multiple alignment program (Thompson, J.D. et al., 1994, Nucleic Acids Res. 22: 4673-4680) with default settings. Alternatively, Clustal W2 and Clustal omega, which are revised versions of Clustal W, can also be used. Clustal W, Clustal W2, and Clustal omega are available on the website of, for example, the European Bioinformatics Institute (EBI [www.ebi.ac.uk/index.html]) or the Japan DNA Data Bank operated by National Institute of Genetics (DDBJ [www.ddbj.nig.ac.jp/searches-j.html]). A position in the target sequence that is aligned to any position in the reference sequence by the above alignment is regarded as the "position corresponding" to any position.

A person skilled in the art can further refine the alignment of the amino acid sequences obtained above to optimize them. Such optimal alignment is preferably determined in consideration of the similarity of amino acid sequences, the frequency of inserted gaps, and the like. The similarity of amino acid sequences as mentioned herein refers to, when two amino acid sequences are aligned, the ratio (%) of the number of positions where the same or similar amino acid residues are present in both sequences to the number of full-length amino acid residues. Similar amino acid residues refer to, among the 20 amino acids constituting a protein, amino acid residues which have similar properties to each other in terms of polarity and charge, and which undergo so-called conservative substitution. A group consisting of such similar amino acid residues is well known to a person skilled in the art, and examples include, but are not limited to, arginine and lysine or glutamine; glutamic acid and aspartic acid or glutamine; serine and threonine or alanine; glutamine and asparagine or arginine; leucine and isoleucine; and the like.

In the present invention, the "parent **α**-amylase" means a standard **α**-amylase to be modified to bring about the mutant of the present invention. The parent may be a natural (wild-type) polypeptide or a mutant thereof.

In the present invention, the amino acid sequence of the parent **α**-amylase or **α**-amylase mutant has at least 85%, preferably at least 90%, more preferably at least 95%, more preferably at least 96%, more preferably at least 97%, more preferably at least 98%, and more preferably at least 99% identity to an amino acid sequence of SEQ ID NO: 4.

The **α**-amylase consisting of the amino acid sequence of SEQ ID NO: 4 is (R178Δ+T180Δ) **α**-amylase mutant having deletion of amino acid residues corresponding to R178 and T180 in an α-amylase consisting of the amino acid sequence of SEQ ID NO: 2 (YR288). A mutant whose parent α-amylase is YR288 and which has deletion of two amino acid residues at positions selected from the group consisting of positions corresponding to positions R178, G179, T180, and G181 in the amino acid sequence of SEQ ID NO: 2 has significantly enhanced stability in cleaning agents, in comparison with YR288 (Patent Literature 6 mentioned above). Therefore, any of **α**-amylase mutants consisting of the amino acid sequence of SEQ ID NO: 2 or an amino acid sequence having at least 90%, preferably at least 95%, more preferably at least 96%, more preferably at least 97%, more preferably at least 98%, and more preferably at least 99% identity thereto and having deletion of two or more amino acid residues selected from positions corresponding to positions R178, G179, T180, and G181 in the amino acid sequence of SEQ ID NO: 2, , as well as an **α**-amylase consisting of the amino acid sequence of SEQ ID NO: 4, can serve as parent **α**-amylases for the mutant of the present invention.

Examples of deletion of amino acid residues at two or more positions preferably include R178Δ+T180Δ, G179Δ+T180Δ, R178Δ+G179Δ, R178Δ+G181Δ, G179Δ+G181Δ, and the like; and more preferably R178Δ+T180Δ.

Other examples of **α**-amylases consisting of an amino acid sequence having at least 90% identity to the amino acid sequence of SEQ ID NO: 4 include DE0178, which is *Bacillus flexus*-derived **α**-amylase, RU2C, which is Bacillus sp.-derived **α**-amylase, and the like (JP-A-2020-121626).

Substitution of amino acid residue at the above predetermined positions means that an amino acid at a position is substituted with a different amino acid.

In the present invention, the number of mutation sites is 2 or more, but is not limited, as long as cleaning performance and/or stability in a protease-containing cleaning agent are ensured. The number of mutation sites may be, for example, 3, 4, 5, 6, 7, or 8, and may be 10 or more. For example, the number of mutation sites can be from 2 to 15, from 2 to 10, or from 3 to 8.

The mutant is preferably an **α**-amylase having at least 90% identity to the amino acid sequence of SEQ ID NO: 4, from the viewpoint of enhancing cleaning performance or stability. The mutant may contain any number of conservative amino acid substitutions as long as it retains the properties as the above mutant.

Preferred embodiments of the substitution of amino acid residues at positions corresponding to positions E187, S241, N192, H240, and K278 of the amino acid sequence of SEQ ID NO: 2 are as shown below.

For example, E187 is preferably substituted with P (E187P);
S241 is preferably substituted with A, Q, D, L, Y, P, or H (S241A/Q/D/L/Y/P/H);
N192 is preferably substituted with F (N192F);
H240 is preferably substituted with F (H240F); and
K278 is preferably substituted with L, D, W, I, H, S, T, N, Q, V, A, Y, or F
   (K278L/D/W/I/H/S/T/N/Q/V/A/Y/F).

Preferred embodiments of the substitution of amino acid residues at positions corresponding to positions F205, R211, and M199 are as shown below.

For example, F205 is preferably substituted with Y (F205Y);
R211 is preferably substituted with L, V, or I (R211L/V/I); and
M199 is preferably substituted with L, T, A, N, Q, S, V, or I (M199L/T/A/N/Q/S/V/I).

Preferred embodiments of the substitution of amino acid residues at positions corresponding to positions G5, S38, Q96, W186, E257, F259, S284, H295, Y296, N303, T320, Y360, W408, L429, V430, G433, A434, W439, N471, G476, and G477 are as shown below.

For example, G5 is preferably substituted with E, D, P, R, or K (G5E/D/P/R/K);
S38 is preferably substituted with N (S38N);
Q96 is preferably substituted with R or K (Q96R/K);
W186 is preferably substituted with L (W186L);
E257 is preferably substituted with T (E257T);
F259 is preferably substituted with W (F259W);
S284 is preferably substituted with W (S284W);
H295 is preferably substituted with Y (H295Y);
Y296 is preferably substituted with A (Y296A);
N303 is preferably substituted with R, E, S, G, V, D, T, or A (N303R/E/S/G/V/D/T/A);
T320 is preferably substituted with D or E (T320D/E);
Y360 is preferably substituted with C, M, L, or V (Y360C/M/L/V);
position W408 is preferably substituted with P (W408P);
L429 is preferably substituted with V (L429V);
V430 is preferably substituted with M (V430M);
G433 is preferably subjected to insertion of S after G (G433GS);
A434 is preferably substituted with V (A434V);
W439 is preferably substituted with R (W439R);
N471 is preferably substituted with T (N471T);
G476 is preferably substituted with A, P, E, S, F, R, or K (G476A/P/E/S/F/R/K); and
G477 is preferably substituted with E (G477E).

Next, the following Table 1-1 shows preferred combinations of mutations consisting of one or more amino acid residue substitutions at positions selected from the group consisting of positions corresponding to positions E187 and S241, and one or more amino acid residue substitutions at positions selected from the group consisting of positions corresponding to positions N192, H240, and K278.

In addition, the following Table 1-2 shows examples of preferred combinations of mutations further combined with one or more amino acid residue substitutions at one or more positions selected from the group consisting of positions corresponding to positions F205, R211, and M199.

**[Table 1-1]**

| |
|---|
| E187+N192F |
| E187P+H240F |
| E187P+K278F |
| E187P+K278Y |
| E187P+K278W |
| H240F+S241Q |
| N192F+H240F+S241Q |

**[Table 1-2]**

| | |
|---|---|
| E187P+N192F+M199L | E187P+M199L+R211I+K278Y |
| E187P+N192F+F205Y | E187P+F205Y+R211I+K278Y |
| E187P+N192F+R211I | E187P+M199L+F205Y+R211I+K278Y |
| E187P+N192F+M199L+F205Y | E187P+M199L+K278W |
| E187P+N192F+M199L+R211I | E187P+F205Y+K278W |
| E187P+N192F+F205Y+R211I | E187P+R2111+K278W |
| E187P+N192F+M199L+F205Y+R211I | E187P+M199L+F205Y+K278W |
| E187P+M199L+H240F | E187P+M199L+R211I+K278W |
| E187P+F205Y+H240F | E187P+F205Y+R211I+K278W |
| E187P+R211I+H240F | E187P+M199L+F205Y+R2111+K278W |
| E187P+M199L+F205Y+H240F | M199L+H240F+S241Q |
| E187P+M199L+R211I+H240F | F205Y+H240F+S241Q |
| E187P+F205Y+R2111+H240F | R211I+H240F+S241Q |
| E187P+M199L+F205Y+R2111+H240F | M199L+F205Y+H240F+S241Q |
| E187P+M199L+K278F | M199L+R211I+H240F+S241Q |
| E187P+F205Y+K278F | F205Y+R211I+H240F+S241Q |
| E187P+R211I+K278F | M199L+F205Y+R211I+H240F+S241Q |
| E187P+M199L+F205Y+K278F | N192F+M199L+H240F+S241Q |
| E187P+M199L+R211I+K278F | N192F+F205Y+H240F+S241Q |
| E187P+F205Y+R211I+K278F | N192F+R211I+H240F+S241Q |
| E187P+M199L+F205Y+R211I+K278F | N192F+M199L+F205Y+H240F+S241Q |
| E187P+M199L+K278Y | N192F+M199L+R211I+H240F+S241Q |
| E187P+F205Y+K278Y | N192F+F205Y+R211I+H240F+S241Q |
| E187P+R211I+K278Y | N192F+M199L+F205Y+R211I+H240F+S241Q |
| E187P+M199L+F205Y+K278Y | |

The mutant of the present invention can be produced by using various mutagenesis techniques known in this technical field. For example, the mutant of the present invention can be produced by mutating a polynucleotide encoding an amino acid residue to be modified in a parent α-amylase gene encoding its standard amino acid sequence (standard α-amylase gene) to a polynucleotide encoding the modified amino acid residue, and then allowing the mutated gene to express a mutant.

The polynucleotide encoding the mutant of the present invention can be in the form of single- or double-stranded DNA, RNA, or an artificial nucleic acid, or may be cDNA or chemically synthesized intron-free DNA.

In the present invention, as the means for mutating an amino acid residue of the parent α-amylase, various mutagenesis techniques known in this technical field can be used. For example, the polynucleotide encoding the mutant of the present invention can be obtained by mutating, in a polynucleotide encoding the amino acid residue of the parent **α**-amylase (hereinafter also referred to as the "parent gene"), a nucleotide sequence encoding an amino acid residue to be mutated to a nucleotide sequence encoding the mutated amino acid residue.

Introduction of the target mutation into the parent gene can be basically performed by various site-specific mutagenesis methods well-known to a person skilled in the art. The site-specific mutagenesis method can be performed by any method, such as an inverse PCR method or an annealing method. It is also possible to use commercially available site-specific mutagenesis kits (e.g., Stratagene's QuickChange II Site-Directed Mutagenesis Kit and QuickChange Multi Site-Directed Mutagenesis Kit, or the like).

Most commonly, site-specific mutagenesis of the parent gene can be performed by using a mutation primer containing the nucleotide mutation to be introduced. The mutation primer may be designed to be annealed to a region containing a nucleotide sequence encoding an amino acid residue to be mutated in the parent gene, and to contain a nucleotide sequence having a nucleotide sequence (codon) encoding the mutated amino acid residue in place of the nucleotide sequence (codon) encoding the amino acid residue to be mutated. The nucleotide sequences (codons) encoding the unmutated or mutated amino acid residues can be appropriately recognized and selected by a person skilled in the art based on ordinary textbooks and the like. Alternatively, site-specific mutagenesis can also be performed by using a method in which DNA fragments, obtained by amplifying the upstream and downstream sides of the mutation site separately using two complementary primers containing the nucleotide mutation to be introduced, are linked into one by SOE (splicing by overlap extension)-PCR (Gene, 1989, 77(1): pp. 61-68).

A template DNA containing the parent gene can be prepared by extracting genome DNA from a microorganism producing the **α**-amylase described above by a standard method, or extracting RNA and synthesizing cDNA with reverse transcription. Alternatively, a corresponding nucleotide sequence may be chemically synthesized based on the amino acid sequence of the parent **α**-amylase, and used as the template DNA. A DNA sequence containing a base sequence encoding an **α**-amylase consisting of the amino acid sequence of SEQ ID NO: 4 is shown in SEQ ID NO: 3, and a DNA sequence containing a base sequence encoding an **α**-amylase consisting of the amino acid sequence of SEQ ID NO: 2 (YR288) is shown in SEQ ID NO: 1.

A mutation primer can be produced by well-known oligonucleotide synthesis methods, such as the phosphoramidite method (Nucleic Acids R4esearch, 1989, 17: 7059-7071). Such primer synthesis can also be performed using, for example, a commercially available oligonucleotide synthesizer (e.g., ABI). Using a primer set containing the mutation primer, site-specific mutagenesis as described above can be carried out using the parent gene as the template DNA, thereby obtaining a polynucleotide encoding the mutant of the present invention having the target mutation.

The polynucleotide encoding the mutant of the present invention can contain single- or double-stranded DNA, cDNA, RNA, or other artificial nucleic acids. The DNA, cDNA, and RNA may be chemically synthesized. The polynucleotide may contain nucleotide sequences of untranslated regions (UTRs) in addition to open reading frames (ORFs). The codon of the polynucleotide may be optimized depending on the species of the transformant used for the production of the mutant polynucleotide of the present invention. Information on codons used by various organisms is available from the Codon Usage Database ([www.kazusa.or.jp/codon/]).

The obtained polynucleotide encoding the mutant of the present invention can be incorporated into a vector. The type of vector to contain the polynucleotide is not particularly limited, and any vector, such as a plasmid, phage, phagemid, cosmid, virus, YAC vector, or shuttle vector, may be used. The vector is not limited, but is preferably a vector which can be amplified in bacteria, preferably Bacillus bacteria (e.g., *Bacillus subtilis* or mutant strains thereof), and more preferably an expression vector which can induce the expression of transgenes in Bacillus bacteria. Among these, shuttle vectors, which are vectors replicable in Bacillus bacteria and any other organisms, can be preferably used in the recombinant production of the mutant of the present invention. Examples of preferred vectors include, but are not limited to, pHA3040SP64, pHSP64R, or pASP64 (JP-B-3492935), pHY300PLK (an expression vector which can transform both *Escherichia coli* and *Bacillus subtilis;* Jpn J Genet, 1985, 60: 235-243), pAC3 (Nucleic Acids Res, 1988, 16: 8732), and other shuttle vectors; pUB110 (J Bacteriol, 1978, 134: 318-329), pTA10607 (Plasmid, 1987, 18: 8-15), and other plasmid vectors which can be used in the transformation of Bacillus bacteria; and the like. Other usable examples include *Escherichia* coli-derived plasmid vectors (e.g., pET22b(+), pBR322, pBR325, pUC57, pUC118, pUC119, pUC18, pUC19, and pBluescript, and the like).

The above vector may contain a DNA replication initiation region or a DNA region containing a replication origin. Alternatively, in the above vector, a regulatory sequence, such as a promoter region for initiating the transcription of the gene, a terminator region, or a secretion signal region for secreting the expressed protein outside the cell, may be operably linked to the upstream of the polynucleotide encoding the mutant of the present invention (i.e., mutant gene). The phrase that a gene and a regulatory sequence are "operably linked" means that the gene and the regulatory region are arranged so that the gene can be expressed under the control of the regulatory region.

The type of regulatory sequence, such as a promoter region, a terminator, or a secretion signal region mentioned above, is not particularly limited, and generally used promoters and secretion signal sequences can be appropriately selected depending on the host for introduction. Examples of preferred regulatory sequences that can be incorporated into the vector include the promoter, secretion signal sequence, and the like of the cellulase gene of Bacllus sp. KSM-S237 strain.

Alternatively, a marker gene (e.g., a gene resistant to drugs, such as ampicillin, neomycin, kanamycin, and chloramphenicol) for selecting the host into which the vector of the present invention is appropriately introduced may be further incorporated into the vector. Alternatively, when an auxotroph is used as the host, a gene encoding the desired nutritional synthetic enzyme may be incorporated as a marker gene into the vector. Alternatively, when a selective culture medium in which a specific metabolism is required for growth, is used, a gene associated with the metabolism may be incorporated as a marker gene into the vector. Examples of such metabolism-related gene include acetamidase genes for utilizing acetamide as a nitrogen source.

The polynucleotide encoding the mutant of the present invention, a regulatory sequence, and a marker gene can be linked by a method known in the art, such as SOE (splicing by overlap extension)-PCR (Gene, 1989, 77: 61-68). Procedures for introducing the linked fragment into the vector are well known in the art.

The transformed cell of the present invention can be obtained by introducing a vector containing the polynucleotide encoding the mutant of the present invention into a host, or by introducing a DNA fragment containing the polynucleotide encoding the mutant of the present invention into the genome of the host.

Examples of the host cells include microorganisms, such as bacteria and filamentous fungi. Examples of bacteria include *Escherichia coli* and bacteria belonging to the genera Staphylococcus, Enterococcus, Listeria, and Bacillus. Preferred among these are *Escherichia coli* and Bacillus bacteria (e.g., *Bacillus subtilis* Marburg No. 168 (*Bacillus subtilis* 168 strain) or mutant strains thereof). Examples of *Bacillus subtilis* mutant strains include the nine-protease-deficient strain KA8AX described in J. Biosci. Bioeng., 2007, 104(2): 135-143, and the eight-protease-deficient strain with improved protein folding efficiency, D8PA strain, described in Biotechnol. Lett., 2011, 33(9): 1847-1852. Examples of filamentous fungi include Trichoderma, Aspergillus, Rizhopus, and the like.

Methods commonly used in the art, such as the protoplast method and the electroporation method, can be used to introduce the vector into the host. Strains with appropriate introduction are selected using marker gene expression, auxotrophy, and the like as indices, whereby the target transformant into which the vector is introduced can be obtained.

Alternatively, a fragment obtained by linking the polynucleotide encoding the mutant of the present invention, a regulatory sequence, and a marker gene can also be introduced directly into the genome of the host. For example, a DNA fragment in which sequences complementary to the genome of the host are added to both ends of the linked fragment is constructed by the SOE-PCR method or the like, and this DNA fragment is then introduced into the host to induce homologous recombination between the host genome and the DNA fragment, whereby the polynucleotide encoding the mutant of the present invention is introduced into the genome of the host.

When the thus-obtained transformant into which the polynucleotide encoding the mutant of the present invention, or a vector containing the polynucleotide is introduced, is cultured in a suitable culture medium, the gene encoding the protein on the vector is expressed to produce the mutant of the present invention. The culture medium used for culturing the transformant can be appropriately selected by a person skilled in the art depending on the type of microorganism of the transformant.

Alternatively, the mutant of the present invention may be expressed from the polynucleotide encoding the mutant of the present invention or a transcript thereof using a cell-free translation system. The "cell-free translation system" is such that reagents, such as amino acids, necessary for the protein translation are added to a suspension obtained by mechanically destroying a cell, which serves as the host, to construct an in vitro transcription-translation system or an in vitro translation system.

The mutant of the present invention produced in the above culture or cell-free translation system can be isolated or purified by using general methods used for protein purification, such as centrifugation, ammonium sulfate precipitation, gel chromatography, ion-exchange chromatography, and affinity chromatography, singly or in a suitable combination. In this case, when the gene encoding the α-amylase mutant of the present invention is operably linked to a secretion signal sequence on the vector in the transformant, the produced protein is secreted extracellularly, and can be thus more easily collected from the culture. The protein collected from the culture may be further purified by known means.

The thus-obtained mutant of the present invention has enhanced cleaning performance and/or stability in comparison with the parent **α**-amylase.

The "enhanced cleaning performance" means an enhanced cleaning effect in comparison with the parent **α-**amylase, for example, an ability to bring about the removal of stains in the washing or cleaning step.

The cleaning performance can be evaluated by using a method well known in the art. For example, a stained cloth cut into a predetermined size is inserted into each well of a 96-well assay plate, and a cleaning agent solution and an enzyme solution are added thereto, followed by cleaning treatment under predetermined conditions. The absorbance at 488 nm of the cleaning liquid after the completion of cleaning is measured, and the difference from the blank ΔA488 is determined as detergency.

The "enhanced stability" means an enhanced ability to maintain **α**-amylase activity in the presence of a cleaning agent containing a protease, in comparison with the parent **α**-amylase.

The stability can be evaluated by using a method well known in the art. For example, an enzyme solution is added to a cleaning agent, the **α**-amylase activity is measured before and after treatment for a predetermined time, the activity value of the sample before treatment is regarded as the initial activity, and the ratio of the activity value after treatment to the initial activity is regarded as the remaining activity (%).

The mutant of the present invention is useful as an enzyme to be contained in various cleaning compositions, and particularly useful as an enzyme to be contained in cleaning compositions suitable for low-temperature cleaning.

Examples of the "low temperature" as mentioned herein include 40°C or lower, 35°C or lower, 30°C or lower, and 25°C or lower, and also include 5°C or higher, 10°C or higher, and 15°C or higher. Other examples include from 5 to 40°C, from 10 to 35°C, from 15 to 30°C, and from 15 to 25°C.

The cleaning composition of the present invention contains the **α**-amylase mutant described above and protease.

The amount of the **α**-amylase mutant contained in the cleaning composition is not particularly limited as long as the protein can exhibit activity. For example, the amount of the **α**-amylase mutant per kg of the cleaning composition is preferably 1 mg or more, more preferably 10 mg or more, and even more preferably 50 mg or more, as well as preferably 5,000 mg or less, more preferably 1,000 mg or less, and even more preferably 500 mg or less. The amount of the α-amylase mutant is also preferably from 1 to 5,000 mg, more preferably from 10 to 1,000 mg, and even more preferably from 50 to 500 mg.

Examples of proteases include proteins having at least 70%, preferably 80% or more, more preferably 90% or more, and more preferably 95% or more identity to the amino acid sequence of SEQ ID NO: 5, 6, 7, 8, 9, 10, or 11, and having protease activity.

The protease consisting of the amino acid sequence of SEQ ID NO: 5 is *Bacillus clausii*-derived protease Savinase (WO 2011/036263), the protease consisting of the amino acid sequence of SEQ ID NO: 6 is *Bacillus amyloliquefaciens*-derived protease BPN' (WO 2011/036263), the protease consisting of the amino acid sequence of SEQ ID NO: 7 is *Bacillus lentus* DSM 5483-derived protease (WO 92/21760), the protease consisting of the amino acid sequence of SEQ ID NO: 8 is Bacillus sp. KSM-KP43-derived protease (WO 99/18218), the protease consisting of the amino acid sequence of SEQ ID NO: 9 is Bacillus sp.-derived protease TY145 (JP-A-2019-503404), the protease consisting of the amino acid sequence of SEQ ID NO: 10 is *Bacillus amyloliquefacience*-derived protease Neutrase, and the protease consisting of the amino acid sequence of SEQ ID NO: 11 is *Bacillus subtilis*-derived metalloprotease.

Further, proteases may be commercially available Alcalase, Esperase, Everlase, Kannase, and Progress Uno (registered trademarks; Novozymes A/S), PREFERENZ, EFFECTENZ, and EXCELLENZ (registered trademarks; DuPont), Lavergy (registered trademark; BASF), and the like.

The amount of the protease contained is not particularly limited, as long as the protein can exhibit activity. For example, the amount of the protease is preferably 1 mg or more, more preferably 10 mg or more, and even more preferably 50 mg or more, and is preferably 5,000 mg or less, more preferably 1,000 mg or less, and even more preferably 500 mg or less, per kg of the cleaning composition. The amount of the protease is also preferably from 1 to 5,000 mg, more preferably from 10 to 1,000 mg, and even more preferably from 50 to 500 mg.

In the cleaning composition, various enzymes other than the above **α**-amylase mutant and protease can be used in combination. Examples of such enzymes include amylases which are different from the above **α**-amylase mutant, cellulases, keratinases, esterases, cutinases, lipases, pullulanases, pectinases, mannanases, glucosidases, glucanases, cholesterol oxidases, peroxidases, laccases, and the like, and preferably cellulases, amylases, and lipases.

Examples of cellulases include Celluclean and Carezyme (registered trademarks; Novozymes A/S); KAC, the alkaline cellulase produced by Bacillus sp. KSM-S237 strain described in JP-A-10-313859, and the mutant alkaline cellulase described in JP-A-2003-313592 (Kao Corporation); and the like.

Examples of amylases include Termamyl, Duramyl, Stainzyme, Stainzyme Plus, and Amplify Prime (registered trademarks; Novozymes A/S), PREFERENZ and EFFECTENZ (registered trademarks; DuPont), KAM (Kao Corporation), and the like.

Examples of lipases include Lipolase and Lipex (registered trademarks; Novozymes A/S), and the like.

Known cleaning agent components can be contained in the cleaning composition of the present invention, and examples of such known cleaning agent components include the following.

### (1) Surfactant

A surfactant may be contained in an amount of from 0.5 to 60 mass% in the cleaning composition, and preferably from 10 to 45 mass% particularly in a powder cleaning composition, and from 20 to 90 mass% in a liquid cleaning composition. When the cleaning composition of the present invention is a clothing cleaning agent for laundry or a cleaning agent for an automatic dishwasher, the surfactant is generally contained in an amount of from 1 to 10 mass%, and preferably from 1 to 5 mass%.

Examples of the surfactant used in the cleaning composition include one or a combination of anionic surfactants, non-ionic surfactants, amphoteric surfactants, and cationic surfactants; and anionic surfactants and non-ionic surfactants are preferred.

Examples of preferred anionic surfactants include sulfate ester salts of alcohols having from 10 to 18 carbon atoms, sulfate ester salts of alkoxylated alcohols having from 8 to 20 carbon atoms, alkylbenzene sulfonate, paraffin sulfonate, **α**-olefin sulfonate, internal olefin sulfonate, **α**-sulfo fatty acid salts, **α**-sulfo fatty acid alkyl ester salts, and fatty acid salts. In the present invention, particularly preferred is one or more anionic surfactants selected from the group consisting of linear alkylbenzene sulfonate with an alkyl chain having from 10 to 14 carbon atoms, more preferably from 12 to 14 carbon atoms, and internal olefin sulfone with an alkylene chain having from 12 to 20 carbon atoms, more preferably from 16 to 18 carbon atoms. Alkali metal salts and amines are preferable as counterions, and sodium and/or potassium, monoethanolamine, and diethanolamine are particularly preferred. For internal olefin sulfonic acid, reference can be made to, for example, WO 2017/098637.

Preferred non-ionic surfactants are polyoxyalkylene alkyl (from 8 to 20 carbon atoms) ether, alkyl polyglycoside, polyoxyalkylene alkyl (from 8 to 20 carbon atoms) phenyl ether, polyoxyalkylene sorbitan fatty acid (from 8 to 22 carbon atoms) ester, polyoxyalkylene glycol fatty acid (from 8 to 22 carbon atoms) ester, and polyoxyethylene polyoxypropylene block polymers. In particular, preferred non-ionic surfactants are polyoxyalkylene alkyl ethers obtained by adding 4 to 20 moles of alkylene oxides, such as ethylene oxide and propylene oxide, to alcohols having from 10 to 18 carbon atoms [an HLB value (calculated by the Griffin method) of from 10.5 to 15.0, and preferably from 11.0 to 14.5].

### (2) Divalent metal ion scavenger

A divalent metal ion scavenger may be contained in an amount of from 0.01 to 50 mass%, and preferably from 5 to 40 mass%. Examples of the divalent metal ion scavenger used in the cleaning composition of the present invention include condensed phosphates, such as tripolyphosphates, pyrophosphates, and orthophosphates; aluminosilicates, such as zeolites; synthetic layered crystalline silicates, nitrilotriacetates, ethylenediaminetetraacetates, citrates, isocitrates, polyacetal carboxylates, and the like. Among these, crystalline aluminosilicates (synthetic zeolites) are particularly preferred. Among A-, X-, and P-type zeolites, A-type zeolites are particularly preferred. As synthetic zeolites, those having an average primary particle size of from 0.1 to 10 µm, particularly from 0.1 to 5 µm, are preferably used.

### (3) Alkali agent

An alkali agent may be contained in an amount of from 0.01 to 80 mass%, preferably from 1 to 40 mass%. In the case of powder cleaning agents, examples of alkali agents include alkali metal carbonates, such as sodium carbonate, collectively called dense ash or light ash; and amorphous alkali metal silicates, such as JIS Nos. 1, 2, and 3. These inorganic alkali agents are effective in the formation of the particle skeleton during cleaning agent drying, and relatively hard cleaning agents having excellent flowability can be obtained. Examples of other alkalis include sodium sesquicarbonate, sodium hydrogencarbonate, and the like. In addition, phosphates, such as tripolyphosphates, also have the action as alkali agents. As alkali agents used in liquid cleaning agents, sodium hydroxide and mono-, di-, or triethanolamine can be used, in addition to the alkali agents mentioned above, and they can also be used as counterions of activators.

### (4) Anti-recontamination agent

An anti-recontamination agent may be contained in an amount of from 0.001 to 10 mass%, preferably from 1 to 5 mass%. Examples of the anti-recontamination agent used in the cleaning composition of the present invention include polyethylene glycol, carboxylic acid-based polymers, polyvinyl alcohol, polyvinylpyrrolidone, and the like. Among these, carboxylic acid-based polymers have the function of scavenging metal ions and the ability to disperse solid particle stains from the clothing into the laundry bath, as well as the anti-recontamination ability. Carboxylic acid-based polymers are homopolymers or copolymers of acrylic acid, methacrylic acid, itaconic acid, or the like. Preferred copolymers are copolymers of the above monomers and maleic acid, and those with a molecular weight of from several thousands to a hundred thousand are preferred. In addition to the carboxylic acid-based polymers mentioned above, polymers such as polyglycidylates, cellulose derivatives such as carboxymethyl cellulose, and amino carboxylic acid-based polymers such as polyaspartic acid are also preferred because they have metal ion scavengers, dispersants, and anti-recontamination ability.

### (5) Bleaching agent

A bleaching agent, such as hydrogen peroxide or percarbonate, may be preferably contained in an amount of from 1 to 10 mass%. When the bleaching agent is used, tetraacetylethylenediamine (TAED) or the bleach activator described in JP-A-6-316700 can be contained in an amount of from 0.01 to 10 mass%.

### (6) Fluorescent agent

Examples of a fluorescent agent used in the cleaning composition include biphenyl-type fluorescent agents (e.g., Tinopal CBS-X and the like) and stilbene-type fluorescent agents (e.g., a DM-type fluorescent dye and the like). The fluorescent agent may be preferably contained in an amount of from 0.001 to 2 mass%.

### (7) Other components

The cleaning composition may contain builders, softeners, reducing agents (e.g., sulfite), foam inhibitors (e.g., silicone), fragrances, antibacterial and antifungal agents (e.g., Proxel [trade name] and benzoic acid), and other additives known in the field of clothing cleaning agents.

The cleaning composition can be produced in accordance with a standard method by combining the **α-**amylase mutant and protease of the present invention obtained by the above method and protease as well as the known cleaning agent components mentioned above. The form of the cleaning agent can be selected depending on the application. For example, the cleaning agent can be in the form of liquid, powder, granules, paste, solids, or the like.

The thus-obtained cleaning composition can be used as a clothing cleaning agent, a dishwashing cleaning agent, a bleaching agent, a cleaning agent for hard surface cleaning, a drain cleaning agent, a denture cleaning agent, a disinfecting cleaning agent for medical instruments, or the like; preferably a clothing cleaning agent and a dishwashing cleaning agent; and more preferably a clothing cleaning agent for laundry (laundry cleaning agent), a dishwashing cleaning agent for hand washing, and a cleaning agent for an automatic dishwasher.

The cleaning composition is suitable for use at 40°C or lower, 35°C or lower, 30°C or lower, or 25°C or lower, and at 5°C or higher, 10°C or higher, or 15°C or higher. The cleaning composition is also suitable for use at from 5 to 40°C, from 10 to 35°C, from 15 to 30°C, or from 15 to 25°C.

Regarding the embodiments described above, the present invention further discloses the following aspects.
<1> A cleaning composition comprising an **α**-amylase mutant and protease, wherein the **α**-amylase mutant is a mutant of a parent **α**-amylase and comprises one or more amino acid residue substitutions at positions selected from the group consisting of positions corresponding to positions E187 and S241 in the amino acid sequence of SEQ ID NO: 2, and further one or more amino acid residue substitutions at positions selected from the group consisting of positions corresponding to positions N192, H240, and K278,
the parent **α**-amylase or **α**-amylase mutant has at least 85% sequence identity to the amino acid sequence of SEQ ID NO: 4.
<2> The cleaning composition according to <1>, wherein the amino acid residue substitutions at positions corresponding to positions E187, S241, N192, H240, and K278 of the **α**-amylase mutant are E187P, S241Q, N192F, H240F, and K278F/Y/W, respectively.
<3> The cleaning composition according to <1> or <2>, wherein the **α**-amylase mutant comprises at least mutations selected from the group consisting of the following combinations a) to g):
   a) E187P+N192F
   b) E187P+H240F
   c) E187P+K278F
   d) E187P+K278Y
   e) E187P+K278W
   f) H240F+S241Q
   g) N192F+H240F+S241Q.
<4> The cleaning composition according to any one of <1> to <3>, wherein the **α**-amylase mutant further comprises one or more amino acid residue substitutions at positions selected from the group consisting of positions corresponding to positions F205, R211, and M199 in the amino acid sequence of SEQ ID NO: 2.
<5> The cleaning composition according to <4>, wherein the amino acid residue substitutions at positions corresponding to positions F205, R211, and M199 of the **α-**amylase mutant are F205Y, R211V/L/I, and M199L/T/A/N/Q/S/V/I, respectively.
<6> The cleaning composition according to <4> or <5>, wherein the **α**-amylase mutant further comprises one or more amino acid residue substitutions at positions selected from the group consisting of positions corresponding to positions G5, S38, Q96, W186, E257, F259, S284, H295, Y296, N303, T320, Y360, W408, L429, V430, G433, A434, W439, N471, G476, and G477 in the amino acid sequence of SEQ ID NO: 2.
<7> The cleaning composition according to <6>, wherein the amino acid residue substitutions at positions corresponding to positions G5, S38, Q96, W186, E257, F259, S284, H295, Y296, N303, T320, Y360, W408, L429, V430, G433, A434, W439, N471, G476, and G477 of the **α-**amylase mutant are G5E/D/P/R/K, S38N, Q96R/K, W186L, E257T, F259W, S284W, H295Y, Y296A, N303R/E/S/G/V/D/T/A, T320D/E, Y360C/M/L/V, W408P, L429V, V430M, G433GS, A434V, W439R, N471T, G476A/P/E/S/F/R/K, and G477E, respectively.
<8> The cleaning composition according to any one of <1> to <7>, wherein the parent **α**-amylase is an **α**-amylase mutant having deletion of amino acid residues at two or more positions, preferably two or more and three or fewer positions, and more preferably two positions selected from the group consisting of positions corresponding to positions R178, G179, T180, and G181 in the amino acid sequence of SEQ ID NO: 2.
<9> The cleaning composition according to <8>, wherein the deletion of amino acid residues at two positions is preferably a combination selected from the group consisting of R178Δ+T180Δ, G179Δ+T180Δ, R178Δ+G179Δ, R178Δ+G181Δ, and G179Δ+G181Δ, and more preferably R178Δ+T180Δ.
<10> The cleaning composition according to any one of <1> to <9>, wherein the protease is a protein having at least 70% identity to the amino acid sequence of SEQ ID NO: 5, 6, 7, 8, 9, 10, or 11 and having protease activity.
<11> The cleaning composition according to any one of <1> to <10>, which is a clothing cleaning agent or a dishwashing cleaning agent.
<12> The cleaning composition according to <11>, which is a clothing cleaning agent for laundry or a dishwashing cleaning agent for hand washing or an automatic dishwasher.
<13> The cleaning composition according to <11> or <12>, which is a powder or a liquid.
<14> The cleaning composition according to any one of <11> to <13>, which is used at a low temperature.
<15> The cleaning composition according to <14>, which is used at 40°C or lower, 35°C or lower, 30°C or lower, or 25°C or lower, and at 5°C or higher, 10°C or higher, or 15°C or higher, or used at from 5 to 40°C, from 10 to 35°C, from 15 to 30°C, or from 15 to 25°C.
<16> The cleaning composition according to <11>, which is used for low-temperature (from 15 to 30°C) cleaning in a laundry, or for low-temperature (from 15 to 30°C) cleaning in an automatic dishwasher.

### Examples

### (1) Construction of YR288 mutant expression plasmid

The method for constructing YR288 mutant described in the following examples is shown below. A forward primer having 15 bases of a sequence complementary to a reverse primer at the 5'-terminal and containing a mutant sequence, and the reverse primer having a base just before the mutant sequence at the 5'-terminal were used as a mutagenesis primer pair. The YR288 R178Δ T180Δ expression plasmid described in the examples of JP-A-2020-121626 or the YR288 mutant expression plasmid produced in this example was used as a template, and PCR was performed by using the mutagenesis primer pair. When multiple fragments were linked, using each of the PCR products, the In-Fusion reaction was performed in accordance with the protocol of the In-Fusion, HD Cloning kit (Clontech). With the PCR product or In-Fusion reaction solution, *Bacillus subtilis* was transformed by the protoplast method to obtain a transformant retaining the target YR288 mutant expression plasmid.

### (2) Enzyme production culture

The recombinant *Bacillus subtilis* colonies obtained in (1) were inoculated in a 96-deep-well plate into which 500 µL of LB culture medium supplemented with 10 ppm tetracycline was dispensed, and then cultured at 32°C at 1,500 rpm overnight. Next day, 20 µL of the culture was inoculated in a 96-deep-well plate into which 500 µL of 2xL-maltose culture medium (2% tryptone, 1% yeast extract, 1% NaCl, 7.5% maltose, 7.5 ppm manganese sulfate pentahydrate, 0.04% calcium chloride dihydrate, and 10 ppm tetracycline; % denotes (w/v)%) was dispensed, and cultured at 32°C at 1,500 rpm for 2 days. Then, the culture supernatant containing the enzyme produced from the bacterial cell was collected by centrifugation and used as an enzyme solution.

### (3) Measurement of protein concentration of culture supernatant

For the measurement of the protein concentration of the culture supernatant, Protein Assay Rapid Kit Wako II (FUJIFILM Wako Pure Chemical Corporation) was used. The protein concentration of a culture supernatant of pHY300PLK (Takara Bio Inc.)-introduced strain without an α-amylase expression cassette was used as a blank to calculate the **α**-amylase concentration of the culture supernatant.

### (4) Evaluation of α-amylase mutant stability (1)

Various mutants were constructed by the method described in Example (1) using YR288 R178Δ+T180Δ (SEQ ID NO: 4) as a parent polypeptide, and the storage stability of these mutants in protease-containing cleaning agents was evaluated.

A commercially available liquid clothing cleaning agent (Kao Corporation, Attack 3X or Lion Corporation, Top Clear Liquid, which are respectively referred to as cleaning agent 1 and cleaning agent 2 in Table 2) was allowed to stand in a boiling water bath for 30 minutes to deactivate the enzyme contained therein. Either protease Savinase (SIGMA, P3111) or KAP8.0Q-L (Kao Corporation) was added to these cleaning agents so that the final concentration was 300 ppm. After mixing well, an amylase solution was added, followed by incubation at 40°C for 2 weeks, and the activity was then measured. The activity value of the sample before the treatment at 40°C was used as the initial activity, and the ratio of the activity value after treatment to the initial activity was regarded as the remaining activity (%). Table 2 shows the results. All of the mutants showed high stability even in any protease-containing cleaning agents.

### (5) Evaluation of α-amylase mutant stability (2)

A commercially available liquid clothing cleaning agent (Kao Corporation, Attack 3X, which is referred to as cleaning agent 1 in Table 3) was allowed to stand in a boiling water bath for 30 minutes to deactivate the enzyme contained therein. Savinase was added to the cleaning agent so that the final concentration was 300 ppm. After mixing well, an amylase solution was added, followed by incubation at 40°C for 2 weeks, and the activity was then measured. The activity value of the sample before the treatment at 40°C was used as the initial activity, and the ratio of the activity value after treatment to the initial activity was regarded as the remaining activity (%). Table 3 shows the results. All of the mutants showed high stability even in the protease-containing cleaning agents.

### (6) Evaluation of amylase detergency after storage in protease-containing cleaning composition

A CS-26 stained cloth cut into a circular shape with a diameter of 5.5 mm was obtained from CFT. Savinase (SIGMA, P3111) and the amylase culture supernatant were added to Attack 3X (treated in a boiling water bath for 30 minutes) so that the final concentration was 300 ppm. After mixing well, incubation was performed at 40°C for 2 weeks, and the resulting samples were subjected to a cleaning test. The CS-26 stained cloth was inserted into each well of a 96-well assay plate, and 200 µL of the protease- and amylase-containing cleaning agent diluted 1,200-fold with tap water was added to each well. The plate was sealed and shaken at 20°C using a Cute Mixer at 1,200 rpm for 15 minutes. After the completion of cleaning, 100 µL of the cleaning liquid was transferred to a new 96-well assay plate, and the absorbance at 488 nm was measured. A blank was prepared by adding ion-exchange water in place of the amylase solution, and the absorbance difference from the blank ΔA488 was determined as α-amylase detergency.

Fig. 1 shows the detergency of the parent **α**-amylase (YR288 R178Δ+T180Δ) and **α**-amylase mutants. The cleaning compositions containing an **α**-amylase mutant and protease maintained excellent detergency at low temperatures, which is a characteristic of **α**-amylase YR288, even after storage at 40°C for 2 weeks, in comparison with the cleaning composition containing the parent **α**-amylase and protease.

**[Table 2-1]**

| Cleaning agent 1+Savinase | Remaining activity (%) |
|---|---|
| R178Δ+T180Δ (Parent enzyme) | 13.7 |
| E187P+N192F | 61.4 |
| E187P+H240F | 70.3 |
| E187P+K278F | 60.5 |
| E187P+K278Y | 62.7 |
| E187P+K278W | 66.2 |
| H240F+S241Q | 71.8 |
| N192F+H240F+S241Q | 76.1 |

**[Table 2-2]**

| Cleaning agent 1+KAP8.0Q-L | Remaining activity (%) |
|---|---|
| R178Δ+T180Δ (Parent enzyme) | 11.8 |
| E187P+N192F | 56 |
| E187P+H240F | 63 |
| E187P+K278F | 57.2 |
| E187P+K278Y | 59.3 |
| E187P+K278W | 58.5 |
| H240F+S241Q | 56 |
| N192F+H240F+S241Q | 67.3 |

**[Table 2-3]**

| Cleaning agent 2+Savinase | Remaining activity (%) |
|---|---|
| R178Δ+T180Δ (Parent enzyme) | 0 |
| E187P+N192F | 91.2 |
| E187P+H240F | 100 |
| E187P+K278F | 84.2 |
| E187P+K278Y | 91.3 |
| E187P+K278W | 84.7 |
| H240F+S241Q | 81 |
| N192F+H240F+S241Q | 88.2 |

**[Table 3-1]**

| Cleaning agent 1+Savinase | Remaining activity (%) |
|---|---|
| R178Δ+T180Δ (Parent enzyme) | 13.1 |
| E187P+N192F+ M199L | 49.7 |
| E187P+N192F+F205Y | 41.3 |
| E187P+N192F+R211I | 47.2 |
| E187P+N192F+M199L+F205Y | 48.1 |
| E187P+N192F+M199L+R211I | 52.2 |
| E187P+N192F+F205Y+R211I | 43.6 |
| E187P+N192F+M199L+F205Y+R211I | 52 |
| E187P+M199L+H240F | 54.4 |
| E187P+F205Y+H240F | 46.6 |
| E187P+R2111+H240F | 45.9 |
| E187P+M199L+F205Y+H240F | 49.6 |
| E187P+M199L+R211I+H240F | 55.2 |
| E187P+F205Y+R211I+H240F | 47.5 |
| E187P+M199L+F205Y+R211I+H240F | 57.2 |
| E187P+F205Y+K278F | 44.6 |
| E187P+R211I+K278F | 46.7 |

**[Table 3-2]**

| Cleaning agent 1+Savinase | Remaining activity (%) |
|---|---|
| R178Δ+T180Δ (Parent enzyme) | 13.7 |
| E187P+M199L+K278F | 57.5 |
| E187P+M199L+F205Y+K278F | 54.5 |
| E187P+M199L+R211I+K278F | 58.4 |
| E187P+F205Y+R211I+K278F | 49.9 |
| E187P+M199L+F205Y+R211I+K278F | 55 |
| E187P+M199L+K278Y | 56.2 |
| E187P+F205Y+K278Y | 46.3 |
| E187P+R211I+K278Y | 49.1 |
| E187P+M199L+F205Y+K278Y | 56.1 |
| E187P+M199L+R211I+K278Y | 57.4 |
| E187P+F205Y+R211I+K278Y | 50.4 |
| E187P+M199L+F205Y+R211I+K278Y | 56.4 |
| E187P+M199L+K278W | 58.3 |
| E187P+F205Y+K278W | 46.3 |
| E187P+R211I+K278W | 51.3 |
| E187P+M199L+F205Y+K278W | 56.2 |

**[Table 3-3]**

| Cleaning agent 1+Savinase | Remaining activity (%) |
|---|---|
| R178Δ+T180Δ (Parent enzyme) | 14.8 |
| E187P+M199L+R211I+K278W | 59.2 |
| E187P+F205Y+R211I+K278W | 49.6 |
| E187P+M199L+F205Y+R211I+K278W | 59.6 |
| M199LH240F+S241Q | 61.3 |
| F205YH240F+S241Q | 54.8 |
| R211IH240F+S241Q | 52.1 |
| M199L+F205Y+H240F+S241Q | 57.9 |
| M199L+R211I+H240F+S241Q | 62.9 |
| F205Y+R211I+H240F+S241Q | 50 |
| M199L+F205Y+R211I+H240F+S241Q | 60.3 |
| N192F+M199L+H240F+S241Q | 64.2 |
| N192F+F205Y+H240F+S241Q | 56.4 |
| N192F+R211I+H240F+S241Q | 57.9 |
| N192F+M199L+F205Y+H240F+S241Q | 62.7 |
| N192F+M199L+R211I+H240F+S241Q | 67.3 |
| N192F+F205Y+R211I+H240F+S241Q | 58.8 |
| N192F+M199L+F205Y+R211I+H240F+S241Q | 67.7 |

## Claims

1. A cleaning composition comprising an **α-**amylase mutant and protease, wherein the **α**-amylase mutant is a mutant of a parent **α**-amylase and comprises one or more amino acid residue substitutions at positions selected from the group consisting of positions corresponding to positions E187 and S241 in the amino acid sequence of SEQ ID NO: 2, and further one or more amino acid residue substitutions at positions selected from the group consisting of positions corresponding to positions N192, H240, and K278,
Wherein the parent **α**-amylase or **α**-amylase mutant has at least 85% sequence identity to the amino acid sequence of SEQ ID NO: 4.

2. The cleaning composition according to claim 1, wherein the amino acid residue substitutions at positions corresponding to positions E187, S241, N192, H240, and K278 of the **α**-amylase mutant are E187P, S241Q, N192F, H240F, and K278F/Y/W, respectively.

3. The cleaning composition according to claim 1 or 2, wherein the **α**-amylase mutant comprises at least mutations selected from the group consisting of the following combinations a) to g):
a) E187P+N192F
b) E187P+H240F
c) E187P+K278F
d) E187P+K278Y
e) E187P+K278W
f) H240F+S241Q, and
g) N192F+H240F+S241Q.

4. The cleaning composition according to any one of claims 1 to 3, wherein the α-amylase mutant further comprises one or more amino acid residue substitutions at positions selected from the group consisting of positions corresponding to positions F205, R211, and M199 in the amino acid sequence of SEQ ID NO: 2.

5. The cleaning composition according to claim 4, wherein the amino acid residue substitutions at positions corresponding to positions F205, R211, and M199 of the α-amylase mutant are F205Y, R211V/L/I, and M199L/T/A/N/Q/S/V/I, respectively.

6. The cleaning composition according to any one of claims 1 to 5, wherein the protease is a protein having at least 70% identity to the amino acid sequence of SEQ ID NO: 5, 6, 7, 8, 9, 10, or 11 and having protease activity.

7. The cleaning composition according to any one of claims 1 to 6, which is a clothing cleaning agent or a dishwashing cleaning agent.

8. The cleaning composition according to claim 7, which is a clothing cleaning agent for laundry or a dishwashing cleaning agent for hand washing or an automatic dishwasher.

9. The cleaning composition according to claim 7 or 8, which is a powder or a liquid.

10. The cleaning composition according to any one of claims 7 to 9, which is used at a low temperature.

11. The cleaning composition according to claim 10, which is used at a temperature of from 5 to 40°C.
